# EUROPEAN PATENT SPECIFICATION

(11) **EP 1 066 833 B1**
(45) Date of publication and mention of the grant of the patent: **01.10.2008**
(21) Application number: 00305764.3
(22) Date of filing: 07.07.2000
(51) Int. Cl.: A61K 35/12, A61K 35/14, A01N 1/02, C12N 5/08, A61P 31/00, A61P 35/00

(54) **Use of cryopreserved activated lymphocytes as a medicament**
Verwendung von kryokonservierten, aktivierten Lymphozyten als Medikament
Utilisation de lymphocytes activés et cryopreservés comme mèdicament

(30) Priority: 08.07.1999 JP 19436299
(43) Date of publication of application: 10.01.2001
(73) Proprietor: Lymphotec Inc., Tokyo (JP)
(72) Inventor: Sekine, Teruaki, Tokyo 135 (JP)
(74) Representative: Neilson, Martin Mark

(56) References cited:
- EP-A- 0 399 647
- WO-A-95/10291
- WO-A-97/39104
- SEKINE T ET AL: "A feasible method for expansion of peripheral blood lymphocytes by culture with immobilized anti-CD3 monoclonal antibody and interleukin-2 for use in adoptive immunotherapy of cancer patients" BIOMEDICINE AND PHARMACOTHERAPY, ELSEVIER, PARIS, FR, vol. 47, no. 2-3, 1993, pages 73-78, XP008007270 ISSN: 0753-3322
- NIERMEIJER M F ET AL: "TRANSPORT AND STORAGE OF AMNIOTIC FLUID SAMPLES FOR PRENATAL DIAGNOSIS OF METABOLIC DISEASES" HUMANGENETIK, SPRINGER VERLAG, BERLIN,, DE, vol. 20, no. 2, 1973, pages 175-178, XP000971556 ISSN: 0018-7348

## Description

This invention relates to cryopreserved cells, a system for producing, processing and supplying the cryopreserved cells by commissioning, and a method for preventing or remedying various types of infections or cancer by using the cryopreserved cells.

Cells are preserved at a low temperature in the state suspended in an adequate preservative solution so as to be thawed when used as required. It is generally thought that the cells do not yet revive to regain their inherent function right after being transformed from the frozen state to the thawed state. To revive the cells after thawed, the thawed cells have been so far cultivated for at least several days, so that the cells thus regaining their function can be used.

In a case of bone marrow transplantation, bone marrow cells collected from a human are usually preserved at a low temperature without being activated in vitro, and then, administered to a patient immediately after being shawed. Besides, effectiveness of the bone marrow administered to the human could not be ascertained until several weeks passed. This is because the bone marrow administered per se has no curing effect, but it is differentiated and proliferated into various sorts of cells to have an organism restored to its wholesome inherent function.

The inventor of this invention, Dr. Teruaki Sekine, has already announced that lymphocytes can be proliferated by solid-phase anti-CD3 antibody and interleukin-2, and autologous lymphocytes thus proliferated have an antitumor effect (Japanese Patent Application Public Disclosure No. HEI 3-80076(A)). The inventor has further announced that the lymphocytes derived from peripheral blood and so forth can be proliferated by the anti-CD3 antibody and interleukin-2, and the autologous lymphocytes have antitumor activity.

There has been reported that autologous lymphocytes proliferated from anti-CD3 antibody and interleukin-2 are effective in the curative treatment of viral infections of a sufferer from congenital immunodefieiciency ("Course of Medicine" by Kiminari Ito and Teruaki Sekine, Vol181, No.6, pp.426-42" (1997)). However, a medical measure as reported is effected by administering cells cultivated to proliferate and dispensed to the organism. Hence, effectiveness of the frozen cells just after thawed has not been verified so far.

Epstein-Barr virus (EBV) is a cancerous virus with which even a human in a healthy condition is latently infected. The healthy human seldom falls into pathogenesis, but an immunological deficient human may be caused to die because there is no sovereign remedy for EBV. Intractable diseases possibly caused by EBV comprehend chronic active EBV infections, which cannot be treated without bone marrow transplantation at present. However, chances of succeeding the bone marrow transplantation are slim, and a marrow donor is not easy to encounter. Furthermore, a patient may be disadvantageously congenial to the bone marrow transplantation due to the unfavorable condition of the health of the patient.

Cellular tissue is generally used for curative treatment just after proliferated and activated, but the proliferation and activation of the cells takes much time to render the application of the cells to symptomatic treatment of acute infections either impossible or very difficult. The cells may be prepared for curative treatment of chronic infections, but it is very difficult to effectively prepare the cells for curative treatment beforehand because a disease by infection is not always contracted.

In effecting the curative treatment of chronic infections, the cells must be prepared every administration, resulting in a rise in expenses of the treatment. In these circumstances, it has been desired to offer a system for stably and speedily supplying the cells for preventing or remedying infections and cancer at a low price.

An object of this invention is to provide medicine consisting of cells as an activated essential for preventing or remedying diseases caused by infections or cancer, which can be produced promptly at a low cost, putting the forthcoming graying age in perspective.

Another object of this invention is to provide a system for stably and promptly mass-producing and supplying cells efficacious against diseases caused by infections or cancer at a low cost and in time for preventing or remedying the diseases.

Still another object of this invention is to provide a method for preventing or remedying various types of infections or cancer by using cells, which can be produced stably and promptly at a low cost.

To attain the objects described above according to this invention, there is provided medicine produced by in vitro proliferating and cultivating or activating and cultivating cells, and freezing the cells so as to thaw the frozen cells thus frozen when putting a human on medication.

The frozen cells can be preserved at an extremely low temperature, namely, cryopreserved. The cryopreserved cells such as lymphocytes obtained by freezing can be suitably used for medical treatment of various infections or cancer. The cryopreserved cells of the invention can be mass-produced and supplied at a low cost in time for preventing or remedying such diseases.

This invention further provides a system for producing, processing and supplying the cryopreserved cells by commissioning, which system comprises a commission section for providing cells in blood, body fluid or tissue, a processing section for proliferating and cultivating or activating and cultivating the cells provided by the commission section, a preservation section for freezing and preserving the cells given by the processing section in their frozen state, and a transport section for supplying the frozen cells to the commission section. According to the system of the invention, the cells efficacious against diseases caused by infections or cancer can be stably mass-produced and promptly supplied at a low cast and in time for preventing or remedying the diseases.

This invention further provides a method for preventing or remedying infections or cancer by using medicine which is produced by in vitro proliferating and cultivating or activating and cultivating cells, and freezing and cryopreserving the cells so as to thaw the cells thus frozen when putting a human on medication.

According to the method of the invention, the cells efficacious against diseases caused by infections or cancer can be stably mass-produced and promptly supplied at a low cost and in time for preventing or remedying the diseases.

One way of carrying out the invention is described in detail below.

The present invention provides cryopreserved cells such as lymphocytes efficacious against diseases caused by infections or cancer, which are obtained by in vitro proliferating and cultivating or activating and cultivating cells generally contained in blood, body fluid or other tissue, and freezing the cells so as to thaw the cells thus frozen when putting a human on medication.

The cells used in this invention may of course be of any cells capable of being in vitro proliferated and cultivated or activated and cultivated. Namely, there may be used not only lymphocytes but also various types of cells such as leucocyte cells, dermal cells, cartilage cells, and bone marrow cells.

The cells can easily be collected, for example, by being extracted from peripheral blood in the easiest collecting method. When collecting the cells from the peripheral blood, it is desirable to gather the cells from venous blood. It is adequate to draw the peripheral blood on the order of 0.01 to 100 *ml* from a vein, but the amount of blood drawn is not specifically limited thereto. Having regard to the burden of a donor, labor of drawing the blood, and operation of separating the cells from the blood, it is desirable to draw 5 to 50 *ml*, more preferably, 10 to 20 *ml* of the peripheral blood at one operation. For preventing coagulation of the blood, heparin or citric acid may be added to the blood drawn from a human.

Separation of the lymphocyte cells from the drawn blood can be effected by a known lymphocyte separating method such as a discontinuous density gradient centrifugation using sucrose or a lymphocyte separating agent on the market.

Proliferation and cultivation of the cells collected from the human can be carried out by a known lymphocyte proliferating method. Although the invention does not contemplate imposing any limitation on the lymphocyte proliferating method, the cells can however be proliferated in the coexistence of interleukin-2 and/or anti-CD3 antibody, as disclosed in Japanese Patent Application Public Disclosure No. HEI 3-80076(A). It is desirable to proliferate the cells in presence of interleukin-2 and anti-CD3 antibody from the standpoint of productivity. For example, the proliferation of the cells may be carried out in such a manner that a culture medium containing interleukin-2, in which the lymphocyte cells are suspended, is placed in a culture vessel containing solid-phase anti-CD3 antibody. The proliferation and activation of the cells may be expedited by using various types of mitogen as required.

Any anti-CD3 antibody capable of expediting the proliferation and activation of the cells may be used, but the type of anti-CD3 antibody is by no means limited in the invention. The anti-CD3 antibody used for stimulating the lymphocyte cells may be yielded in an animal or cell by use of refined CD3 molecules, while there may be effectively used OKT-3 antibody, which is put on the market and available stably and inexpensively (made by Ortho Pharmaceutical Corp.)

From the standpoint of efficiency of proliferating and handling the lymphocyte cells, it is desirable to use the anti-CD3 antibody in its solid state. To solidify the antibody, a culture vessel made of glass, polyurethane, polyolefine, or polystyrene may be used adequately. A sterilized cell-culture flask of plastic is availably on the market, while the size of the flask is not specifically limited in the invention.

Solidification of the anti-CD3 antibody can be effected by placing a diluen: of the anti-CD3 antibody into a solidifying vessel and letting it to stand undisturbed at a temperature of 40°C to 37°C for 2 to 20 hours. For the solidification of the anti-CD3 antibodies, the anti-CD3 antibodies are desired to be diluted in a physiological buffer solution such as a sterilized dulbecco phosphate buffer solution to the concentration of 1 to 30 *µg*/*ml*.

After solidifying, the antibodies can be preserved in a cold room or refrigerator (at 4°C) until using. When the solution is removed, solidified antibodies may be rinsed with the physiological buffer solution such as the dulbecco phosphate buffer solution at a normal temperature, if need arises.

It is further desirable for practicing the invention to use interleukin-2 with the culture medium solution to increase the efficiency of proliferating the lymphocyte cells. The interleukin-2 is desirably used at concentration of 1 to 2000 *U*/*ml* in the culture medium solution. The interleukin-2 on the market may be used adequately. The interleukin-2 is used in the state dissolved in water, physiological saline, dulbecco phosphate buffer solution or a culture solution for cultivating cells, such as RPMI-1640, DMEM, IMDM, and AIM-V, which have been widely used in general. The interleukin-2 once dissolved in the culture solution is required to be kept cold during storage in order for preventing degradation in activity.

As the culture medium solution for cultivating the desired cells, there may be used a culture medium derived from a living organism or a culture medium composed by mixing amino acid, vitamins, nucleic acid base and so on with equilibrium salt solution, but these should be understood as limitative as far as the culture medium applied to the invention is suitable for cultivating the cells. For example, as the culture medium, RPMI-1640, AIM-V, DMEM, IMDM or the like can be used preferably. In particular, the culture medium of RPMI-1640 is most recommendable. The culture medium with addition of normal human serum excels in proliferating effect and can be preferably used. Thees culture mediums applicable to the invention have been on the market.

The cultivation of the desired cells may be fulfilled by a common cell-cultivating method. For example, it can be carried out in a CO² incubator at a CO²-concentration of 1% to 10%, preferably 5%, at a temperature of 30°C to 40°C, preferably 37°C in particular.

The cells cultivated are preserved in the frozen state at an extremely low temperature, i.e. cryopreserved, in the following manner. The concentration of the cells suspended in a preservative solution may be arbitrarily determined in accordance with the size of the cell, but it is desirable to suspend the cells in the preservative solution in concentration from 1 × 10³/*ml* to 1 × 10¹⁰/*ml*, preferably, 1 × 10⁵/*ml* to 1 × 10⁸/*ml* in order to preserve the frozen cells. The quantity of the solution containing the cells to be preserved is by no means limited, but it may be determined in the range of 0.1 *ml* to 1000 *ml* for convenience, preferably 0.5 *ml* to 100 *ml*.

To preserve the cells in the frozen state, there may be used not only a marketing preservative solution, but also an in-house preservative solution. There may be used a usual preservative solution which contains any suitable buffer solution, or is made by adding serum or protein, polymer substance such as polysaccharide and dimethylsulfoxide (DMSO) to a basic culture medium, but these constituents are not absolutely necessary to the preservative solution according to the cells to be cryopreserved. Thus, the cells suspended in the suitable preservative solution can be preserved in the frozen state.

The temperature at which the cells are cryopreserved may be arbitrarily determined according to the type of the cells and the properties of the preservative solution, but the cells are generally preserved at a temperature below 0°C. In a case of preparing the frozen cells in ampuls, there may be preserved 1 to 1000 ampuls, preferably 2 to 100 ampuls in one lot so as to thaw the cells in the ampuls by one lot in use.

The system according to the invention, which serves for producing, processing, supplying and using the cryopreserved cells by commissioning for medicinal purposes, comprises a commission section for providing cells contained in blood, body fluid or tissue, a processing section for proliferating and cultivating or activating and cultivating the cells provided by the commission section, a preservation section for freezing and preserving the cells given by the processing section in their frozen state, and a transport section for supplying the frozen cells to the commission section.

The commission section in this system includes a physician, dentist, medical organ, patient, patient's family, and other concerns requiring or processing cells. The commission section may provide blood, body fluid or tissue containing cells. The processing section to which the commission section gives the cells contained in the blood, body fluid or tissue serves to proliferate or activate the given cells. The processing section includes medical organ and other concerns for processing, proliferating or activating the cells. The preservation section includes medical organ and concerns for cryopreserving the cells processed by the processing section. The transport section for transporting the cells cryopreserved from the preservation section to the commission section is the so-called transport traders.

The commission section, processing section, preservation section and transport section may be independent of one another, or all or some of them may be merged in one section or organization. That is to say, in a case that the cells proliferated and cultivated or activated and cultivated in vitro by the processing section are frozen by the preservation section, the cells may be cryopreserved by the preservation section or commission section. While the cells may be frozen and cryopreserved by the commission section, the commission section may commission the preservation section to cryopreserve the frozen cells. Thus, the processing section or preservation section may provide the cells in the frozen state or thawed state to the commission section (client requesting preservation of the cells).

The cells preserved in the frozen state according to the invention can be administered to a human immediately after being thawed for preventing or remedying various diseases, as described above. The cells reviving by being thawed may be further proliferated and cultivated or activated and cultivated if need arises. The reviving cells prepared just after thawed take effect on various diseases including not only viral infections due to immunodeficiency or immunosuppression, but also all sorts of infections and cancer. Specifically, the reviving cells according to this invention are efficacious against a disease, which must be urgently treated.

### [EMBODIMENT 1]

### (1) Separation of lymphocytes:

20 *ml* of peripheral blood was drawn from the vein of a patient contracting chronic active EBV infection, adding heparin thereto. A syringe needle of a syringe with which the blood was collected aseptically in a clean bench (S-1100 model made by Showa Science Co., Ltd.) was removed from a barrel of the syringe without touching a joint portion between the needle and barrel of the syringe, and replaced with another 19G ×11/2" syringe needle made by Nipro Co., Ltd.

Into two centrifugation tubes of 50 *ml* (Product No. 2341-050 made by Iwaki Glass Co., Ltd.), a rinsing culture medium having a capacity of 500 *ml* (RPMI1640+6) (Product No. GM1106 made by Nikken Bio Medical Laboratory) was placed by 15 *ml*, and then, all the blood collected was poured equally at a slow speed.

After tightly close the centrifugation tubes with lids, the contents in the tubes were mingled by turning the tubes upside down two to three times. Then, 3 *ml* of Lymphosepar-I (Product No. 23010 made by Immuno Biological Laboratory) was poured into each of six centrifugation tubes having a capacity of 15 *ml* (Product No. 2327-015 made by Iwaki Glass Co., Ltd.) by using a pipette having a capacity of 10 *ml* (Product No. 4105 imported and marketed by Corning Costar Japan Corp.), and then, 10 *ml* of blood diluted with the culture medium was slowly poured into each of centrifugation tubes so as not to disarrange the surface of the contents in each tube.

The centrifugation tubes thus prepared were mounted in a centrifugal separator (H-700R made by Kokusan Corp.) and subjected to centrifugal separation at 1800 rpm at a centrifugal temperature of 20°C for 15 minutes in the state of switching off a brake.

After subjecting the centrifugal separation, lymphocyte cells thus separated were slowly sucked up to the height of about 1 *cm* from a layer of lymphocytes in the centrifugation tube by use of an evacuator in the asepsis state, taking care not to be sucked out. Then, the layer of lymphocyte cells was collected with a pipette of 5 *ml* in capacity, taking care not to suck a layer of blood clots, to the centrifugation tube of 50 *ml* in capacity into which 25 *ml* of rinsing culture medium (RPMI1640+6) was placed in advance. The centrifugation tube closed with a lid was turned upside down two to three times, and then, subjected to centrifugal separation at 1800 rpm at a centrifugal temperature of 20°C for 10 minutes.

Upon completion of the centrifugal separation, supernatant liquid in the centrifugation tube was removed, and sedimentum of cells was stirred by using a vortex to be thoroughly loosened.

Next, suspension in which the cells are suspended was prepared by mixing and well stirring 44 *ml* of medium (PRPMI1640+7 made by Immuno Biological Laboratory), 50 *ml* of cultivating culture medium including 1 *ml* of 35,000 *U*/*ml* IL-2 made by Cetus Corporation and 5 *ml* of human serum. 10 *µl* of the cell suspension was placed in a tube (Model 72.690 imported and marketed by Assist Co., Ltd.) and mixed with 40 *µl* of Turk's solution made by Muto Pure Chemicals Co., Ltd. Then, 10 *µl* of the mixture thus obtained was applied to a hemocytometer (Model 9731 made by Perkin-Elmer Corp.) and observed by a microscope (Model 211320 made by Olympus Optical Co., Ltd.) to count the cells. Consequently, 2.0 × 10⁷ of cells in total were counted out.

### (2) Preparation of OKT3-solidifying flask:

Into a culture flask having a bottom area of 225 *cm²* (Product No. MS-2080R made by Sumitomo Bakelite Co., Ltd.), 10 *ml* of OKT3 solution (produced by Ortho Pharmaceutical Corp. and imported and marketed by Janssen-Kyowa Co., Ltd.) prepared with PBS(-) to 5 *µg*/*ml* was poured, uniformly flooding the bottom of the flask. The OKT3 solution thus prepared flasks was sucked up by using an evacuator the next day. Upon pouring 50 *ml* of PBS(-) into the flask, the flask closed with a lid was hard shaken, and thereafter, upon opening the lid, the solution was thrown away. Again, upon pouring 50 *ml* of PBS(-) into the flask in the clean bench, the flask was closed with the lid and hard shaken, and thereafter, upon opening the lid, the solution was thrown away. Then, the remaining solution in the flask was carefully sucked up by using the evacuator. In the aforementioned manner, the OKT3-solidifying flask was prepared

### (3) Cultivation of lymphocytes to activate:

The aforementioned cell-suspended solution was inoculated into the OKT3-solidifying flask prepared in the process (2) mentioned above and cultivated in the atmosphere of 5% of carbonic acid gas in saturated humidity at 37°C After 3 days, upon adding 50 *ml* of culture solution thereto, the cells in the suspension were cultivated in the atmosphere of 5% of carbonic acid gas in saturated humidity at 37°C. Further, after 4 days, upon adding 150 *ml* of culture solution thereto, the cells were cultivated in the atmosphere of 5% of carbonic acid gas in saturated humidity at 37°C. Further, the cells were cultivated in the atmosphere of 5% of carbonic acid gas in saturated humidity at 37°C for 2 days, to obtain 3 × 10⁸ of activated lymphocytes.

### (4) Cryopreservation of activated lymphocytes:

After 3 days, the activated lymphocytes obtained in the aforementioned process (3) were subjected to centrifugal separation to remove the cultivating culture medium by decantation, consequently to obtain the cell pellets. To the cell pellets, there was added 5 *ml* of cell-preserving solution prepared by mixing 5 *ml* of human serum, 18 *ml* of dimethyl sulfoxide (called "DMSO" made by Nakarai Tesque Inc.) and 40 *ml* of culture medium solution (RPMI1640+7). The mixture thus obtained was well mingled and equally parted into five cell-preserving tubes (Product No. 430289 imported and marketed by Corning Costar Japan Corp.) by 3 *ml* (5 × 10⁷ per tube). The tubes were preserved in a cryogenic freezer at -80°C.

### (5) Thawing and revitalization of cryopreserved cells:

After 3 days, one of the tubes cryopreserved in the aforementioned process (4) was taken out and warmed at 37°C for 4 minutes by a heat block Model TAL-1G made by Taitec Corp.) to permit the frozen cells to thaw and revive. 3 *ml* of cell-preserving solution containing the thawed and revived cells was aseptically poured into a centrifugation tube having a capacity of 15 *ml*, and 10 *ml* of physiological saline was added thereto, to suspend the cells in the solution. After subjecting the solution to centrifugal separation at 1000 rpm at 20°C for 5 minutes, supernatant liquid was removed by decantation, and 10 *ml* of physiological saline was further added thereto.

Further, the solution was subjected to centrifugal separation at 1000 rpm at 20°C for 5 minutes to remove supernatant liquid by decantation, and 10 *ml* of physiological saline was further added to suspend the cells in the solution. Again, the solution was subjected to centrifugal separation at 1000 rpm at 20°C for 5 minutes to remove supernatant liquid by decantation, and 10 *ml* of physiological saline containing 5%-human seralbumin solution was further added to suspend the cells in the solution, thus obtaining medicine to be administered.

### (6) Administration of medicine:

The medicine obtained in the aforementioned process (5) was infused into the vein of the patient contracting chronic active EBV infection, whose bk od was collected. The fact that, a few days later, a marked improvement in clinical symptoms such as reduction of hepatosplenometaly could be confirmed, revealed that the cryopreserved cells according to this invention can be effectively used for preventing or remedying various types of infections.

### [EMBODIMENT 2]

### (1) Administration of medicine:

The frozen cells prepared in Embodiment 1 noted above were thawed and revived in the same manner as Embodiment 1 to obtain medicine to be administered to a patient. The medicine thus obtained was administered to the same patient as in Embodiment 1 two weeks after providing the medicine for the patient in Embodiment 1. After administration of the medicine, the condition of the patient was coming along well. As a consequent, this fact revealed the cryopreserved cells according to the invention can be continuously used effectively for preventing or remedying various types of infections.

As is apparent from the foregoing description, according to the present invention, the medicine produced by in vitro proliferating and cultivating or activating and cultivating cells, and freezing the cells can be arbitrarily thawed when putting a human on medication. The medicine according to the invention takes effect on various diseases including not only viral infections due to immunodeficiency or immunosuppression, but also all sorts of infections, cancer, infections caused by EBV, i.e. tumor virus. Further, the medicine according to the invention has an excellent effect on prevention and remedy for various allergies, systemic lupus erythematosus (SLE), rheumatoid arthritis (RA), Sjogren syndrome, myasthenia gravis, pernicious anemia (PA), and autoimmune diseases such as Hashimoto disease.

Moreover, the cryopreserved cells according to the invention can be effectively used for preventing or remedying bronchial asthma, cryptomeria pollinosis, urticaria and other various allergic diseases. While the medicine of the invention can be effectively used for preventing or remedying various diseases including viral infections due to immunodeficiency or immunosuppression, all sorts of infections, and cancer as described above, the cryopreserved cells according to this invention are specifically effectual for preventing or remedying a disease which must be urgently treated, because the frozen cells of the invention can be used immediately after thawed.

The medicine according to the invention can be stably mass-produced and supplied at a low cost as many as need be by the system for producing, processing, supplying and using the cryopreserved cells by commissioning for medicinal purposes, which comprises the commission section for providing cells contained in blood, body fluid or tissue, the processing section for proliferating and cultivating or activating and cultivating the cells provided by the commission section, the preservation section for freezing and preserving the cells given by the processing section in their frozen state, and the transport section for supplying the frozen cells to the commission section.

The preventing or remedying method according to the invention can be applied to not only a human, but also an animal such as a dog, cat, cattle, pig sheep, and horse.

## Claims

1. Use of cryopreserved activated lymphocytes in the manufacture of a medicament for preventing or remedying infections and cancer, **characterised in that** said lymphocytes are produced by activating and cultivating lymphocytes *in vitro* in the presence of anti-CD3 antibody and interleukin-2, and which lymphocytes are then cryopreserved in a preservative solution before being thawed for immediate administration to a patient.

2. Use of cryopreserved activated lymphocytes according to claim 1, **characterised in that** said anti-CD3 antibody is solidified.

## Patentansprüche

1. Verwendung von kryokonservierten aktivierten Lymphozyten bei der Herstellung eines Medikaments zur Prävention oder Heilung von Infektionen und Krebs, **dadurch gekennzeichnet, dass** die Lymphozyten hergestellt werden durch Aktivieren und Kultivieren von Lymphozyten *in vitro* in der Gegenwart von anti-CD3-Antikörper und Interleukin-2, und wobei die Lymphozyten anschließend in einer konservierenden Lösung kryokonserviert werden, bevor sie für eine sofortige Verabreichung an einen Patienten aufgetaut werden.

2. Verwendung von kryokonservierten aktivierten Lymphozyten nach Anspruch 1, **dadurch gekennzeichnet, dass** der anti-CD3-Antikörper verfestigt ist.

## Revendications

1. Utilisation de lymphocytes activés cryoconservés dans la fabrication d'un médicament pour prévenir ou guérir les infections et le cancer, **caractérisée en ce que** les lymphocytes sont produits en activant et en cultivant des lymphocytes *in vitro* en présence d'anticorps anti-CD3 et d'interleukin-2, ces lymphocytes étant ensuite cryoconservés dans une solution de conservation avant d'être décongelés pour être immédiatement administrés à un patient.

2. Utilisation de lymphocytes activés cryoconservés selon la revendication 1, **caractérisée en ce que** l'anticorps anti-CD3 est solidifié.
